**Europäisches Patentamt**

**European Patent Office**

**Office européen des brevets**

(19)

(11) Veröffentlichungsnummer : **0 520 940 A1**

## (12) EUROPÄISCHE PATENTANMELDUNG

(21) Anmeldenummer : **92810411.6**

(22) Anmeldetag : **27.05.92**

(51) Int. Cl.⁵ : **A61F 2/46**

(30) Priorität : **26.06.91 CH 1888/91**

(43) Veröffentlichungstag der Anmeldung :
**30.12.92 Patentblatt 92/53**

(84) Benannte Vertragsstaaten :
**AT CH DE FR GB IT LI**

(71) Anmelder : **SULZER Medizinaltechnik AG**
**Fröschenweidstrasse 10**
**CH-8404 Winterthur (CH)**

(72) Erfinder : **Willert, Hans Georg, Prof. Dr. Med.**
**Schlegelweg 9**
**W-3400 Göttingen (DE)**
Erfinder : **Koch, Rudolf**
**Rosenhuben**
**CH-8500 Frauenfeld (CH)**

(74) Vertreter : **Hammer, Bruno, Dr.**
**c/o Gebrüder Sulzer AG, KSR/Patente/0007**
**CH-8401 Winterthur (CH)**

(54) **Ausschlaginstrument für Hüftgelenkprotesenschäfte.**

(57)     Die Erfindung zeigt Ausschlaginstrumente für Hüftgelenkprothesenschäfte, die aus einem Schaft (1) mit Kupplungsteil zu einem Gleithammer (nicht gezeigt) sowie aus einem Ansatzstück (3), welches in Ausschlagrichtung ansetzbar ist, bestehen. Das Ansatzstück (3) ist starr mit dem Schaft verbunden und steht als Fuss von diesem in einem Winkel $\alpha$ zwischen 20° und 60° weg. Das Ansatzstück weist eine Aussparung (8) auf, die sich einseitig über den Hals einer Hüftgelenkprothese schieben lässt und diesen auf einem Winkel von 180° umgibt, wobei die über 180° gemessene lichte Weite der Aussparung (8) um ein Spiel grösser ist als der in der gleichen Richtung gemessene kleinste Durchmesser des Prothesenhalses.

Fig. 1

Die Erfindung handelt von einem Ausschlaginstrument für Hüftgelenkprothesenschäfte bestehend aus einem Schaft mit Kupplungsteil zu einem Gleithammer sowie aus einem Ansatzstück, das in Ausschlagrichtung ansetzbar ist.

Das Ausschlagen der Schäfte von Hüftgelenkprothesen ist bei Reoperationen unumgänglich, um das Knochenbett für einen neuen Schaft vorzubereiten. Die Schaftprothesen weisen daher oft Löcher oder Gewindebohrungen im proximalen Teil des Prothesenschaftes auf, an denen ein Ausziehwerkzeug angesetzt werden kann, welches über ein Kupplungsteil mit einem Hammer verbunden ist, derart, dass der Schaft über Schläge in Ausziehrichtung wegen der Trägheit des betroffenen Beins ausgezogen wird. Ein derartiges Ausschlaginstrument ist in EP 0 244 610 gezeigt. Ein als Finger endender Haken greift dort in eine Bohrung ein, die quer zur Achse des Prothesenschaftes liegt. Ein Nachteil der bisherigen Ausführungen ist, dass an dem Prothesenschaft Löcher oder Kupplungsmöglichkeiten vorgesehen werden müssen, die sich mit Körpergewebe zusetzen, die für ihre Zugänglichkeit einen in Auszugsrichtung vorstehenden Prothesenschaft bedingen, die die Prothese eventuell schwächen und die bei zementfreier Verankerung die mögliche Angriffsfläche für ein Einschlagwerkzeug verringern.

Hier schafft die Erfindung Abhilfe. Sie löst die Aufgabe, ein starres, einteiliges Ausschlaginstrument an einer Femurkopfprothese anzusetzen, für dessen Anbringen keinerlei Rücksicht in der Prothesenkonstruktion notwendig ist.

Gemäss der Erfindung wird die Aufgabe dadurch gelöst, dass das Ansatzstück starr mit dem Schaft verbunden ist, dass das Ansatzstück als Fuss in einem Winkel $\alpha$ zwischen 20° und 60° vom Schaft wegsteht und dass das Ansatzstück eine Aussparung aufweist, die sich einseitig über den Hals des Prothesenschaftes einer Hüftgelenkprothese schieben lässt und diesen auf einem Winkel von 180° umgibt, wobei die über die 180° gemessene lichte Weite der Aussparung um ein Spiel grösser ist als der in der gleichen Richtung gemessene kleinste Durchmesser des Halses.

Die Vorteile der Erfindung sind darin zu sehen, dass ein einfaches robustes Ausziehwerkzeug zu Verfügung steht, welches ohne Vorbereitungsaufwand zum Ausschlagen an der Prothese angesetzt werden kann. Die abhängigen Ansprüche 2 bis 7 beziehen sich auf weitere vorteilhafte Ausbildungen der Erfindung.

Im folgenden wird die Erfindung anhand von Ausführungsbeispielen beschrieben. Es zeigen:

Fig. 1 die Seitenansicht eines erfindungsgemässen Ausziehwerkzeuges, welches über den Kugelkopf der Prothese einführbar ist;

Fig. 2 eine um 90° versetzte Seitenansicht mit Teilschnitt der Anordnung in Figur 1, die an einer Prothese angesetzt ist;

Fig. 3 eine Anordnung nach Figur 2 mit einem durch einen Wulst erzeugten tiefsten Berührungspunkt am Prothesenhals;

Fig. 4 die Seitenansicht eines seitlich über den Prothesenhals schiebbaren Ausschlagwerkzeuges;

Fig. 5 eine um 90° versetzte Seitenansicht der Anordnung in Figur 4, die an eine Prothese angesetzt ist.

Die Figuren zeigen Ausschlaginstrumente für Hüftgelenkprothesenschäfte, die aus einem Schaft 1 mit Kupplungsteil 2 zu einem Gleithammer (nicht gezeigt) sowie aus einem Ansatzstück 3, welches in Ausschlagrichtung ansetzbar ist, bestehen. Das Ansatzstück 3 ist starr mit dem Schaft verbunden und steht als Fuss von diesem in einem Winkel $\alpha$ zwischen 20° und 60° weg. Das Ansatzstück weist eine Aussparung 8 auf, die sich einseitig über den Hals einer Hüftgelenkprothese schieben lässt und diesen auf einem Winkel von 180° umgibt, wobei die über 180° gemessene lichte Weite der Aussparung 8 um ein Spiel grösser ist als der in der gleichen Richtung gemessene kleinste Durchmesser des Prothesenhalses.

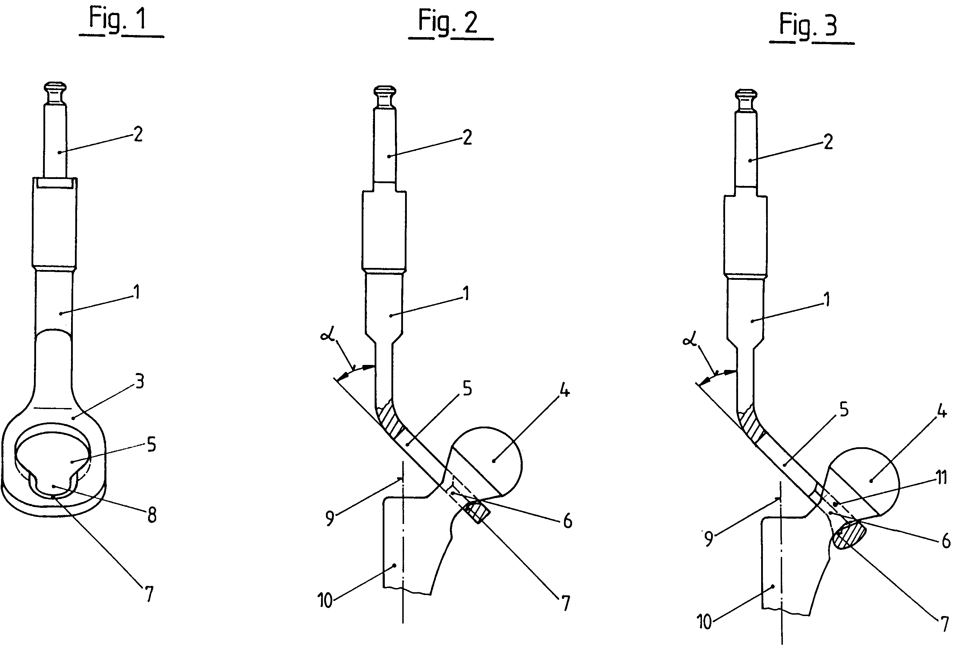

In Figur 1, 2 und 3 ist ein einteiliges starres Ausschlaginstrument gezeigt, das aus einem Schaft 1 und in der Schaftachse fortgesetzt aus einem Kupplungsteil 2 besteht, an dem ein Gleithammer (nicht gezeigt) fest angekoppelt werden kann.

Ein in sich ebenes Ansatzstück 3 steht in einem Winkel $\alpha$ zwischen 20° und 60° von der Schaftachse weg und bildet einen geschlossenen Ring mit einem Durchbruch 5, dessen kleinster Durchmesser grösser als der Durchmesser der Hüftgelenkkugel 4 ist. An seinem unteren Ende ist der Durchbruch 5 um eine Aussparung 8 erweitert, in die der engste Querschnitt der Prothesenhals 6 bei Bewegungen in der Ebene des Ansatzstückes 3 von medial gerade noch einfahrbar ist, bis der Prothesenhals 6 über einen Winkel von 180° von der Aussparung 8 umgeben ist. Ein Zug in Längsrichtung von Schaftachse 1 bewirkt, dass die Aussparung 8 in einem tiefsten Punkt 7 und an zwei um 180° versetzten Punkten 11, welche auf Höhe der Halsmitte zum Kugelkopf hin liegen, anliegt. Durch Anbringen von einem Wulst wie in Figur 3 kann der tiefste Punkt 7 einen noch grösseren Abstand zu den beiden Auflagepunkten 11 einnehmen, um mit den beiden Auflagepunkten 11 den Prothesenhals noch besser in der Aussparung 8 zu verklemmen und ein Reaktionsmoment zu bilden, welches das Moment, das durch Kraftangriff ausserhalb der Schaftachse 9 des Prothesenschafts 10 entsteht, kompensiert. Wenn der Schaft 1 des Ausschlaginstrumentes in dieser Klemmlage in die Schaftachse 9 der Prothesenschäfte 10 zu liegen kommt, entsteht über die Prothese eine reine Zugbelastung auf das Knochenbett in Richtung der Schaftachse 9. Der Scherwirkung zwischen Prothesenoberfläche und Knochenbett werden keine zusätzlichen Spannungen überlagert, die die Festigkeit des Knochens gefährden.

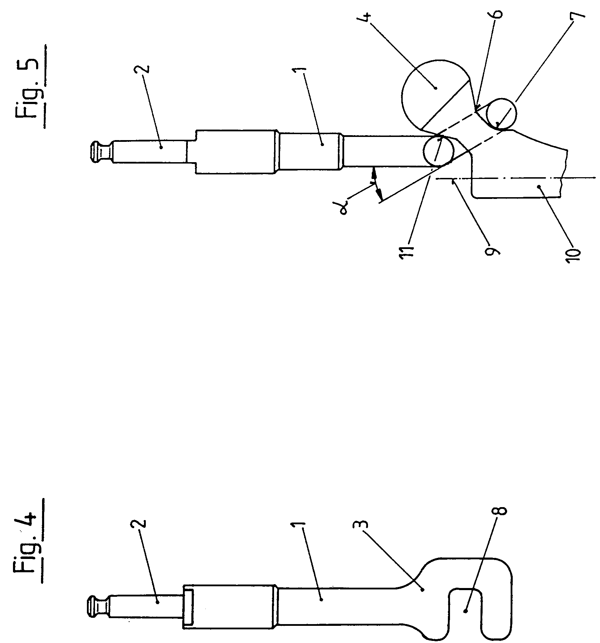

In den Figuren 4 und 5 ist im Ansatzstück 3, das ebenfalls in einem Winkel α zwischen 20° und 60° vom Schaft 1 des Ausziehinstrumentes wegsteht, eine von aussen zugängliche seitliche Aussparung 8 angebracht, die sich seitlich von dorsal oder ventral über den engsten querschnitt des Prothesenhalses schieben lässt. Bei Zugbelastung am Ausziehinstrument wird zwischen dem tiefsten Punkt 7 und einem höchsten Auflagepunkt 11 durch Klemmen ein Reaktionsmoment am Prothesenhals 6 erzeugt, das dem Moment für Kraftangriff ausserhalb der Schaftachse 9 des Prothesenschaftes 10 entgegenwirkt.

In beiden Ausführungsformen fährt der Operateur die Aussparung 8 über den Prothesenhals und setzt das Ausziehinstrument unter leichte Zugspannung bis durch geringes Abkippen der Ausziehvorrichtung eine Klemmung am Prothesenhals 6 eintritt. Unter Beibehaltung der leichten Vorspannung, die die Klemmung und starre Verbindung bezüglich Zugkraft sicherstellt, werden Ausziehschläge mit einem angekoppelten Gleithammer erzeugt, um den Prothesenschaft von seinem Knochenbett zu trennen.

## Patentansprüche

1. Ausschlaginstrument für Hüftgelenkprothesenschäfte bestehend aus einem Schaft (1) mit Kupplungsteil (2) zu einem Gleithammer sowie aus einem Ansatzstück (3), das in Ausschlagrichtung ansetzbar ist, **dadurch gekennzeichnet,** dass das Ansatzstück (3) starr mit dem Schaft (1) verbunden ist, dass das Ansatzstück (3) als Fuss in einem Winkel α zwischen 20° und 60° vom Schaft (1) wegsteht und dass das Ansatzstück eine Aussparung (8) aufweist, die sich einseitig über den Hals (6) des Prothesenschaftes (10) einer Hüftgelenkprothese schieben lässt und diesen auf einem Winkel von 180° umgibt, wobei die über die 180° gemessene lichte Weite der Aussparung (8) um ein Spiel grösser ist als der in der gleichen Richtung gemessene kleinste Durchmesser des Halses (6).

2. Ausschlaginstrument nach Anspruch 1, dadurch gekennzeichnet, dass die Aussparung (8) im Ansatzstück (3) an einen Durchbruch (5) angrenzt, dessen Durchmesser grösser als der Durchmesser der Hüftgelenkkugel (4) ist.

3. Ausschlaginstrument nach Anspruch 2, dadurch gekennzeichnet, dass der Hals (6) des Prothesenschaftes (10) vollständig in der Aussparung (8) eintaucht und am tiefsten Punkt (7) auf einem angeformten Wulst das Ansatzstück (3) berührt.

4. Ausschlaginstrument nach Anspruch 3, dadurch gekennzeichnet, dass bei annähernd parallel zur Achse (9) des Prothesenschaftes (10) ausgerichtetem Schaft (1), die Aussparung (8) in zwei um 180° versetzten Punkten (11) und am tiefsten Punkt (7) den Hals (6) berührt, wobei die drei Punkte (11, 7) annähernd in einer Ebene parallel zur Schaftachse (9) der Prothese liegen, um bei Zugbelastung im Schaft (1) ein Moment zwischen Ansatzstück (3) und Prothesenhals (6) zu übertragen.

5. Ausschlaginstrument nach Anspruch 1, dadurch gekennzeichnet, dass die Aussparung (8) quer zur Achse vom Schaft (1) und von aussen zugänglich im Ansatzstück (3) angebracht ist.

6. Ausschlaginstrument nach Anspruch 5, dadurch gekennzeichnet, dass bei annähernd parallel zur Achse (9) des Prothesenschaftes (10) ausgerichtetem Schaft (1) die Aussparung (8) in einem tiefsten Punkt (7) und in einem höchsten Punkt (11) den Hals (6) berührt, um bei Zugbelastung im Schaft (1) ein Moment zwischen Ansatzstück (3) und Prothesenhals (6) zu übertragen.

7. Ausschlaginstrument nach einem der Ansprüche 1 bis 6, dadurch gekennzeichnet, dass während dem Ausschlagen der Schaft (1) in der Achse (9) vom Prothesenschaft (10) liegt.

Fig. 1  Fig. 2  Fig. 3

EP 0 520 940 A1

Fig. 5

Fig. 4

**Europäisches Patentamt**

# EUROPÄISCHER RECHERCHENBERICHT

Nummer der Anmeldung

EP 92 81 0411

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y<br>A | US-A-4 222 382 (ANTONSSON)<br>* Spalte 2, Zeile 3 - Zeile 60;<br>Abbildungen *<br>--- | 1,2,5<br>3,4 | A61F2/46 |
| Y<br>A | US-A-3 955 568 (NEUFELD)<br>* Spalte 2, Zeile 11 - Spalte 3, Zeile 14;<br>Abbildungen *<br>--- | 1,2,5<br>6 | |
| A | FR-A-2 615 097 (LANDOS)<br>* Zusammenfassung; Abbildungen *<br>--- | 1,3,4,7 | |
| A | EP-A-0 175 079 (WALDEMAR LINK)<br>--- | | |
| D,A | EP-A-0 244 610 (SULZER)<br>----- | | |
| | | | **RECHERCHIERTE SACHGEBIETE (Int. Cl.5)**<br><br>A61F |

Der vorliegende Recherchenbericht wurde für alle Patentansprüche erstellt

| Recherchenort | Abschlußdatum der Recherche | Prüfer |
|---|---|---|
| DEN HAAG | 02 OKTOBER 1992 | KLEIN C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes Dokument

EPO FORM 1503 03.82 (P0403)